# EUROPEAN PATENT SPECIFICATION

(11) **EP 3 922 310 B1**
(45) Date of publication and mention of the grant of the patent: **08.04.2026**
(21) Application number: 20752028.9
(22) Date of filing: 07.02.2020
(51) Int. Cl.: A61P 9/10, A61P 9/14, A61P 41/00, A61P 43/00, A61L 31/16, A61K 35/28, A61L 27/38

(54) **PHARMACEUTICAL COMPOSITION FOR PREVENTING IN-STENT RESTENOSIS**
PHARMAZEUTISCHE ZUSAMMENSETZUNG ZUR VORBEUGUNG VON IN-STENT-RESTENOSE
COMPOSITION PHARMACEUTIQUE DESTINÉE À PRÉVENIR LA RESTÉNOSE INTRA-STENT

(30) Priority: 07.02.2019 JP 2019020707
(43) Date of publication of application: 15.12.2021
(73) Proprietor: Sapporo Medical University, Sapporo-shi, Hokkaido 060-8556 (JP); Nipro Corporation, Settsu-shi, Osaka 566-8510 (JP)
(72) Inventor: HONMOU Osamu, Sapporo-shi Hokkaido 060-8556 (JP); SASAKI Masanori, Sapporo-shi Hokkaido 060-8556 (JP); SASAKI Yuko, Sapporo-shi Hokkaido 060-8556 (JP); OKA Shinichi, Sapporo-shi Hokkaido 060-8556 (JP); NAKAZAKI Masahito, Sapporo-shi Hokkaido 060-8556 (JP); MAEZAWA Rie, Sapporo-shi Hokkaido 060-8556 (JP)
(74) Representative: Hoffmann Eitle
(86) International application number: PCT/JP2020/004698
(87) International publication number: WO 2020/162580

(56) References cited:
- WO-A1-2005/097147
- NAKAZAKI MASAHITO ET AL: "Prevention of neointimal hyperplasia induced by an endovascular stent via intravenous infusion of mesenchymal stem cells", vol. 133, no. 6, 1 December 2020 (2020-12-01), US, pages 1773 - 1785, XP055970437, ISSN: 0022-3085, Retrieved from the Internet <URL:http://dx.doi.org/10.3171/2019.7.JNS19575> DOI: 10.3171/2019.7.JNS19575
- KIM AE-KYEONG ET AL: "Inhibitory effects of mesenchymal stem cells in intimal hyperplasia after balloon angioplasty", JOURNAL OF VASCULAR SURGERY, ELSEVIER, AMSTERDAM, NL, vol. 63, no. 2, 18 September 2014 (2014-09-18), pages 510 - 517, XP029392977, ISSN: 0741-5214, DOI: 10.1016/J.JVS.2014.08.058
- AMALIA FORTE ET AL: "Stem Cell Therapy for Arterial Restenosis: Potential Parameters Contributing to the Success of Bone Marrow-Derived Mesenchymal Stromal Cells", CARDIOVASCULAR DRUGS AND THERAPY, KLUWER ACADEMIC PUBLISHERS, BOSTON; US, vol. 26, no. 1, 15 December 2011 (2011-12-15), pages 9 - 21, XP035019037, ISSN: 1573-7241, DOI: 10.1007/S10557-011-6359-8
- CHANG HYUN-KYUNG ET AL: "Coronary stents with inducible VEGF/HGF-secreting UCB-MSCs reduced restenosis and increased re-endothelialization in a swine model", vol. 50, no. 9, 1 September 2018 (2018-09-01), KR, pages 1 - 14, XP055970444, ISSN: 1226-3613, Retrieved from the Internet <URL:http://www.nature.com/articles/s12276-018-0143-9.pdf> DOI: 10.1038/s12276-018-0143-9
- OSAMU HONMOU ET AL: "Intravenous administration of auto serum-expanded autologous mesenchymal stem cells in stroke", BRAIN, vol. 134, no. 6, 14 April 2011 (2011-04-14), GB, pages 1790 - 1807, XP055253698, ISSN: 0006-8950, DOI: 10.1093/brain/awr063
- PETER J PSALTIS: "Mechanistic Insights into Arterial Repair with Mesenchymal Stromal Cells ; Editorial to: "Stem Cell Therapy for Arterial Restenosis: Potential Parameters Contributing to the Success of Bone Marrow-Derived Mesenchymal Stromal Cells" by A. Forte et al", CARDIOVASCULAR DRUGS AND THERAPY, KLUWER ACADEMIC PUBLISHERS, BOSTON; US, vol. 26, no. 1, 13 December 2011 (2011-12-13), pages 1 - 3, XP035019036, ISSN: 1573-7241, DOI: 10.1007/S10557-011-6362-0
- KENICHI TAMAMA ET AL: "Epidermal Growth Factor (EGF) Treatment on Multipotential Stromal Cells (MSCs). Possible Enhancement of Therapeutic Potential of MSC", JOURNAL OF BIOMEDICINE AND BIOTECHNOLOGY, vol. 2010, 1 January 2010 (2010-01-01), pages 1 - 10, XP055451518, ISSN: 1110-7243, DOI: 10.1155/2010/795385
- CUI LI-LI ET AL: "Integrin [alpha]4 Overexpression on Rat Mesenchymal Stem Cells Enhances Transmigration and Reduces Cerebral Embolism After Intracarotid Injection", STROKE, vol. 48, no. 10, 1 October 2017 (2017-10-01), US, pages 2895 - 2900, XP93146441, ISSN: 0039-2499, DOI: 10.1161/STROKEAHA.117.017809
- ZHANG, G. W. ET AL.: "Mechanisms of the protective effects of BMSCs promoted by TMDR with heparinized bFGF-incorporated stent in pig model of acute myocardial ischemia", J. CELL . MOL. MED., vol. 15, no. 5, 2011, pages 1075 - 1086, XP055729939
- JIN, G. Z. ET AL.: "IMPLANTATION OF STENT COATED WITH ANTIBODY AGAINST MESENCHYMAL STEM CELLS PREVENTS RESTENOSIS", ACTA MEDICA MEDITERRANEA, vol. 29, 2013, pages 785 - 789, XP055842025
- LI, X. ET AL.: "Effects of Human Umbilical Cord Mesenchymal Stem Cell Therapy on CD 61, CD 62P and CD 54 in Elderly Patients with Old Myocardial Infarction", JOURNAL OF THE AMERICAN GERIATRICS SOCIETY, vol. 30, no. 3, 2013, pages 157, XP055842031
- WU, X. ET AL.: "Mesenchymal stem cell seeding promotes reendothelialization of the endovascular stent", J. BIOMED MATER RES PART A, vol. 98 A, no. 3, 2011, pages 442 - 449, XP055729941

## Description

### Technical Field

### Related application

The present specification includes the contents described in the specification of Japanese Patent Application No. 2019-020707 (applied on February 7, 2019), based on which the present application claims priority.

### Technical field

The present invention relates to a pharmaceutical composition comprising mesenchymal stem cells for use in a method for preventing in-stent restenosis, wherein the pharmaceutical composition is intravenously administered to a patient subjected to stenting, wherein 5 x 10⁷ or more mesenchymal stem cells per dose are administered.

### Background Art

In carotid artery stenting (CAS) or percutaneous transluminal angioplasty and stenting (PTAS) implemented in the treatment of carotid artery or intracranial atherosclerosis, postoperative in-stent restenosis is a critical complication which leads to the recurrence of stroke (cerebral infarction). Although it has been reported that the risk of ipsilateral recurrence of cerebral infarction after CAS is low, medium or more severe restenosis occurs more frequently than endarterectomy after PTAS, and restenosis increases the risks of ipsilateral recurrence of stroke and transient ischemic attack (TIA). Despite improvement in operative technique, in-stent restenosis after the PTAS of intracranial atherosclerosis has also been reported as a serious complication, and the incidence was 31% at highest. Stenosis causes stroke or TIA in 39% of the patients. Generally speaking, these findings emphasize that in-stent restenosis after PTAS needs to be prevented to prevent ipsilateral ischemic stroke or TIA and improve clinical results of diseases after CAS and PTAS.

Possible mechanism of in-stent restenosis is neointimal hyperplasia induced by inflammatory response to stent struts (Non Patent Literatures 1 and 2). In cardiology, drug-eluting stents (DESs) have been developed to reduce the restenosis rate by preventing neointimal hyperplasia through suppressing inflammatory response. The off label use of DESs for intracranial artery disease has also been attempted, however, the periprocedural complication rate was high because of technical failure due to stent stiffness and neurotoxicity. Therefore, the development of a new approach for inhibiting neointimal hyperplasia has been desired.

It has been reported that the intravenous administration of mesenchymal stem cells (MSCs) derived from bone marrow reduces reperfusion injury and stroke volume and improves behavioral function in an experimental stroke model (Patent Literatures 1 to 5). Suggested mechanisms from recent studies include the stabilization of inflammatory microenvironments through the release of various anti-inflammation mediators, in addition to another therapeutic mechanisms of MSCs such as neuroprotection, neurogenesis, induction of axonal sprouting, angiogenesis, restoration of blood brain barrier, remyelination, the preservation of cortical connection, and the secretion of neurotrophic factors which impart neural plasticity and remote response(Non Patent Literatures 3 to 7) (Non Patent Literature 8). WO 2005/097147 A1 discloses restenosis therapy using mesenchymal stem cells.

### Citation List

### Patent Literature

Patent Literature 1: WO 2002/000849
Patent Literature 2: WO 2009/034708
Patent Literature 3: WO 2018/034023
Patent Literature 4: WO 2018/034314
Patent Literature 5: WO 2017/188457

### Non Patent Literature

Non Patent Literature 1: Dussaillant GR, et al: Small stent size and intimal hyperplasia contribute to restenosis: a volumetric intravascular ultrasound analysis. J Am Coll Cardiol 26:720-724, 1995
Non Patent Literature 2: Hoffmann R, et al: Patterns and mechanisms of in-stent restenosis. A serial intravascular ultrasound study. Circulation 94: 1247-1254, 1996
Non Patent Literature 3: Lankford KL, et al: Intravenously delivered mesenchymal stem cell-derived exosomes target M2-type macrophages in the injured spinal cord. PLoS One 13:e0190358, 2018
Non Patent Literature 4: Nakazaki M, et al: Intravenous infusion of mesenchymal stem cells inhibits intracranial hemorrhage after recombinant tissue plasminogen activator therapy for transient middle cerebral artery occlusion in rats. J Neurosurg 127:917-926, 2017
Non Patent Literature 5: Prockop DJ, et al: Mesenchymal stem/stromal cells (MSCs): role as guardians of inflammation. Mol Ther 20:14-20, 2012
Non Patent Literature 6: Shi Y, et al: Immunoregulatory mechanisms of mesenchymal stem and stromal cells in inflammatory diseases. Nat Rev Nephrol, 2018
Non Patent Literature 7: Sasaki Y, et al: Synergic Effects of Rehabilitation and Intravenous Infusion of Mesenchymal Stem Cells After Stroke in Rats. Phys Ther 96:1791-1798, 2016

### Summary of Invention

Any references to methods for treatment of the human or animal body by surgery or therapy in the context of the present invention in this description are to be interpreted as references to pharmaceutical compositions of the present invention for use in those methods.

### Technical Problem

An object of the present invention as defined in the claims is to provide a pharmaceutical composition for use in a method for preventing restenosis after PTAS and the recurrence of stroke or TIA accompanying it.

### Solution to Problem

The inventors built up the hypothesis that the intravenous administration of MSC would inhibit neointimal hyperplasia by suppressing the inflammatory reaction to the implanted stent struts using a porcine model. The inventors developed two types of bare metal stents in mini-pigs. One stent was implanted at the common carotid artery (CCA) corresponding to the human middle cerebral artery (MCA), and the other stent was implanted into the superficial cervical artery (SCA) corresponding to the human CCA. Image analysis using angiography (DSA) and intravascular ultrasonography (IVUS) were performed. Histological findings including inflammatory changes around the stent struts after MSC administration were evaluated. The inventors confirmed that MSCs can also be administered to a patient subjected to stenting, and the progress of neointimal hyperplasia could, moreover, be suppressed in the CCA and the SCA subjected to stenting thereby.

The present invention has been completed based on the above-mentioned findings and is set out in the appened set of claims.

### Advantageous Effects of Invention

According to the present invention as defined in the claims, a pharmaceutical composition comprising mesenchymal stem cells for use in method for preventing in-stent restenosis, as defined in the claims, is provided. The pharmaceutical composition for use according to the present invention as defined in the claims can prevent in-stent restenosis after PTAS, and can prevent the recurrence of stroke and the occurrence of TIA. It is known that mesenchymal stem cells have the effect of accelerating the regeneration of the damaged site in ischemic angiopathy such as myocardial infarction and cerebral infarction to improve motor dysfunction. It is also known that mesenchymal stem cells have the effect of treating dementia, cerebral infarction in the chronic phase, spinal cord injury in the chronic phase, neurodegenerative disease, mental disease, higher-order functional disorder, and the like by the effect of forming synapses and promoting the plasticity in a damaged site (a lesion part). Therefore, the pharmaceutical composition for use according to the present invention as defined in the claims can be expected to prevent in-stent restenosis and have a preferable effect also on the above-mentioned symptoms.

### Brief Description of Drawings

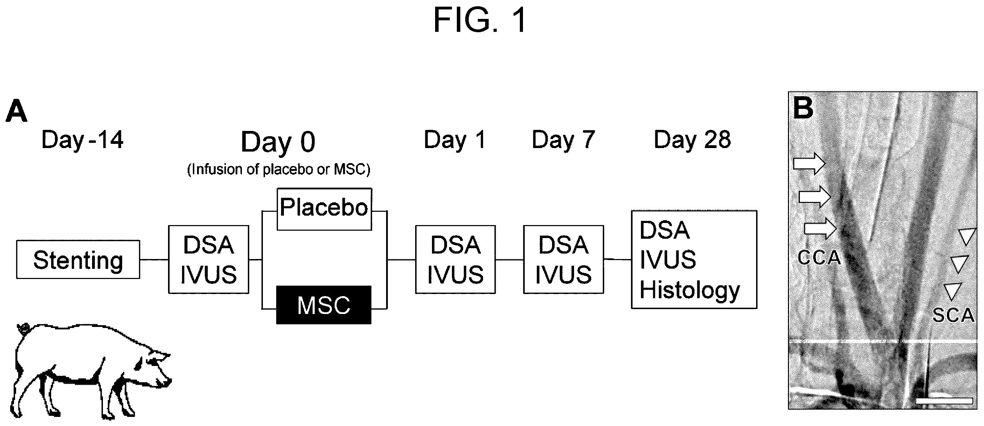

[Figure 1] Figure 1 shows an experiment protocol of the Examples. (A) Mini-pigs are randomized into a placebo administration group and a MSC administration group 14 days after stent implantation, and a placebo or MSCs (1.0 × 10⁸ cells each) are intravenously administered. Angiography (DSA) and intravascular ultrasonography (IVUS) are performed immediately before administration and at day 1, day 7, and day 28. (B) shows an angiograph of stented vessels and the contralateral common carotid artery (control) after the evaluation of day 28(the arrows indicate the CCA and the SCA).

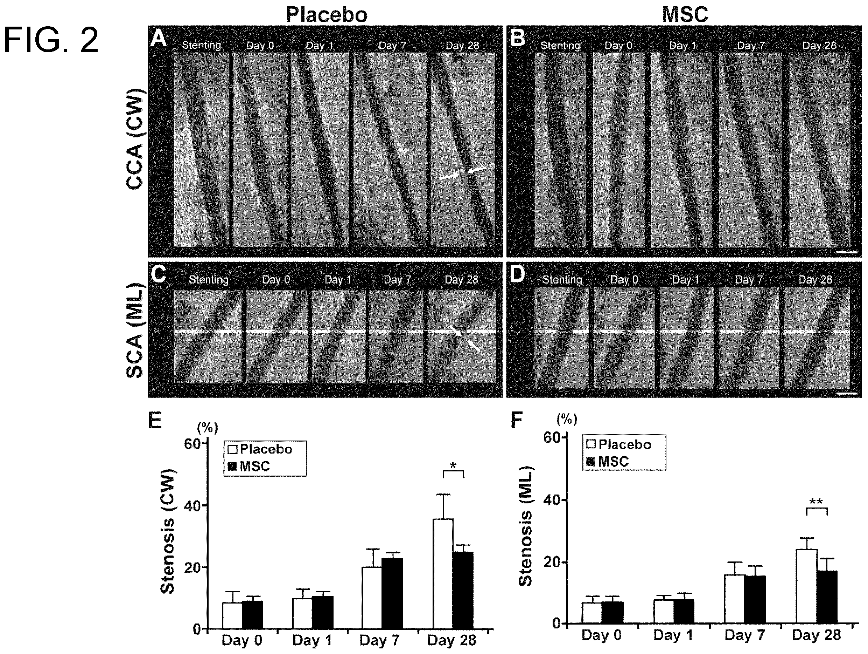

[Figure 2] Figure 2 shows the results of angiography. A, B, C, and D show the CCAs stented with CW (A: placebo administration group, B: MSC administration group) and the SCAs stented with ML (C: placebo administration group, D: MSC administration group). E and F show the results obtained by quantifying stenosis (stenosis rates) (E: CCA stented with CW, F: SCA stented with ML). Scale bars = 4 mm (A, B), 1 mm (C, D). *p < 0.05, **P < 0.01 (CW: Carotid WALLSTENT^{®}, ML: Multi-Link 8^{®}).

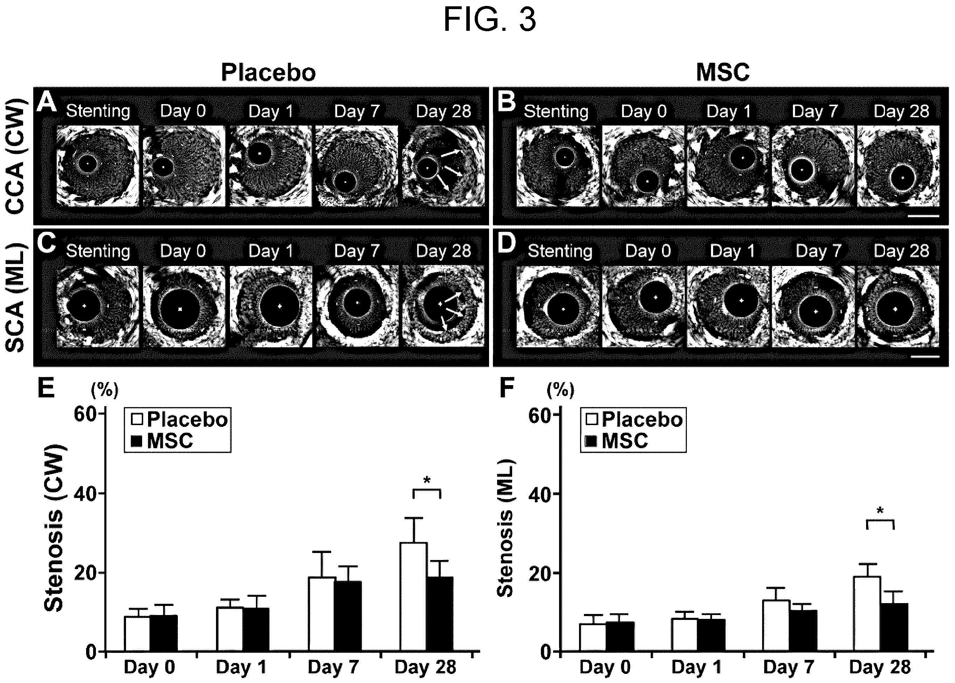

[Figure 3] Figure 3 shows results of IVUS. A, B, C, and D show the CCAs stented with CW (A: placebo administration group, B: MSC administration group) and the SCAs stented with ML (C: placebo administration group, D:MSC administration group). E and F show the results obtained by quantifying stenosis (stenosis rates) (E: CCA stented with CW, F: SCA stented with ML). Scale bars = 2 mm (A, B), 1 mm (C, D). *p < 0.05

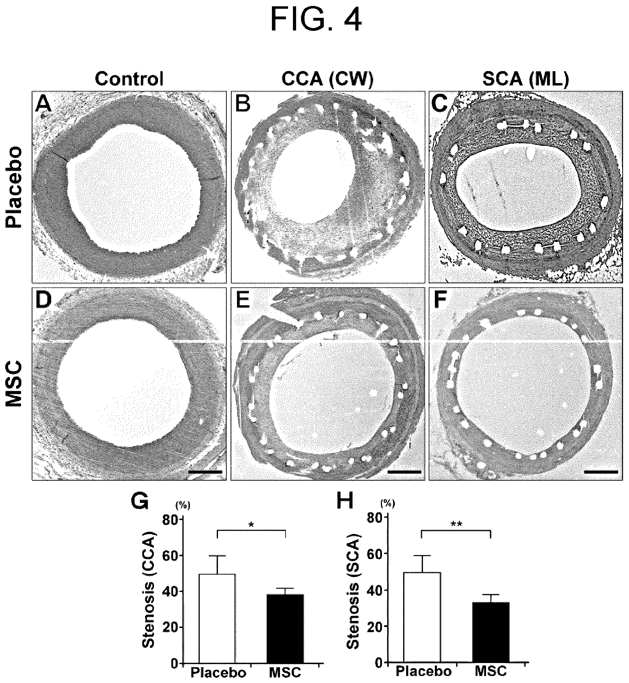

[Figure 4] Figure 4 shows the results of HE staining (microscopic images at low magnification). A to F show control arteries (A: placebo administration group, D:MSC administration group), the CCAs (B: placebo administration group, E: MSC administration group), and the SCAs (C: placebo administration group, F: MSC administration group). G and H show the results obtained by quantifying stenosis (stenosis rates) (G: CCA stented with CW, H: SCA stented with ML). Scale bars = 10 mm (A, B, D, E), 5 mm (C, F). *p < 0.05, **P < 0.01

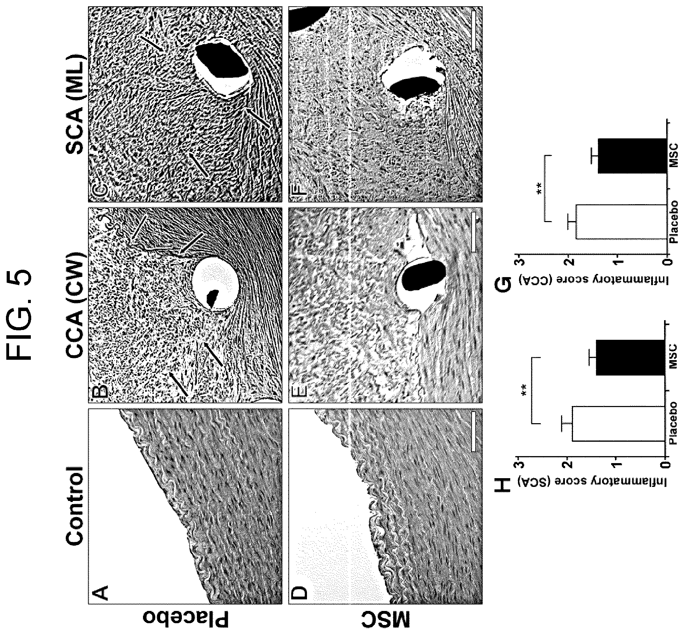

[Figure 5] Figure 5 shows the results of HE staining (microscopic images at high magnification). A to F show control arteries (A: placebo administration group, D:MSC administration group), the CCAs (B: placebo administration group, E: MSC administration group), and the SCAs (C: placebo administration group, F: MSC administration group). G and H show the results obtained by quantifying stenosis (stenosis rate) (G: CCA stented with CW, H: SCA stented with ML). Scale bars = 10 mm (A, D), 5 mm (B, C, E, F). **P < 0.01

### Description of Embodiments

The present invention as defined in the claims relates to a pharmaceutical composition comprising mesenchymal stem cells for use in a method for preventing in-stent restenosis, as defined in the claims.

### 1. Mesenchymal stem cells

The "mesenchymal stem cells" used in the pharmaceutical composition for use according to the present invention as defined in the claims are stem cells which exist in interstitial cells in mesenchymal tissue in a very small amount and has pluripotency and self-replication ability, and it is known that such cells not only differentiate into connective tissue cells such as bone cells, cartilage cells, and adipocytes, but also have the ability to differentiate into neurons and cardiac muscle cells.

The source of the mesenchymal stem cells may be cells differentiation-induced from ES cells or induced pluripotent stem cells (iPS cells), cells from established cell lines, or cells isolated from the living body and proliferated. Although examples of the source in the living body include bone marrow, peripheral blood, umbilical cord blood, and the brain, mesenchymal stem cells derived from bone marrow or blood, especially bone marrow mesenchymal stem cells, particularly human bone marrow mesenchymal stem cells are preferable. Mesenchymal stem cells derived from bone marrow are advantageous in that 1) a remarkable effect can be expected, 2) the risk of side effects is low, 3) enough donor cells can be expected to be supplied, 4) since administering mesenchymal stem cells is noninvasive treatment, and mesenchymal stem cells can be autotransplanted, and thus mesenchymal stem cells have advantages including the following points: 5) low risk of infectious disease; 6) no concern about immunorejection; and 7) no ethical problem; 8) easier social acceptance and 9) ease of broader establishment as a general medical treatment. The bone marrow transplantation therapy is treatment which has already been used at clinical sites, and the safety is also confirmed. Stem cells derived from bone marrow are highly migratory, stem cells reach target damaged tissue not only by local transplantation but also by intravenous administration, and a therapeutic effect can be expected.

Although the cells may be derived from allogeneic cells or autologous cells, mesenchymal stem cells derived from autologous cells (derived from a patient's own cells) are preferable.

Mesenchymal stem cells used in the present invention as defined in the claims are preferably undifferentiated. It is because undifferentiated cells have a high reproductive rate and a high survival rate after introduction into the living body. The undifferentiated state can be confirmed, for example, by confirming that CD24, which is a differentiation marker, is not expressed. The inventors have also developed a method for obtaining such cells, and its details are described in International Publication No. WO 2009/002503.

In the method developed by the inventors, cells separated from bone marrow fluid or the like under the conditions where the cells are substantially out of contact with an anticoagulant (heparin or the like) are proliferated using culture medium containing homologous serum (preferably autologous serum; human serum in the case of a pharmaceutical composition for use in humans) and containing no anticoagulant (heparin or the like) or an anticoagulant at a very low concentration. "Containing no anticoagulant (heparin or the like) or an anticoagulant at a very low concentration" means not containing an anticoagulant in an effective amount as an anticoagulant. For example, in the case of heparin or its derivative, the effective amount as an anticoagulant is usually around 20 to 40 µg/mL. In a method developed by the inventors, when the amount beforehand added to a blood-collecting tube for sample collection is minimized, the amount in a sample collected from the living body is less than 5 U/mL, preferably less than 2 U/mL, and more preferably less than 0.2 U/mL, and the amount existing in the culture medium when the cells are cultured is less than 0.5 U/mL, preferably less than 0.2 U/mL, and more preferably less than 0.02 U/mL based on the volume of the culture medium (refer to International Publication No. WO 2009/034708) .

The density of cells in the culture medium affects properties of cells and the direction of the differentiation. In the case of mesenchymal stem cells, when the cell density in the culture medium is more than 8,500 cells/cm², properties of the cells change, and the cells are therefore preferably subcultured at a cell density of at most 8,500 cells/cm² or less and more preferably subcultured when the cell density is 5,500 cells/cm² or more.

Since a human serum-containing culture medium is used in the method developed by the inventors, the culture medium is desirably exchanged as few times as possible in view of the burden of a serum donor, and the culture medium is exchanged, for example, at least once a week, more preferably 1 to 2 times a week.

As to the culture, the cells are subcultured repeatedly until the total number of the cells is 10⁸ or more. Although the number of required cells can vary depending on the purpose, for example, the number of mesenchymal stem cells required for transplantation for use in the treatment of ischemic encephalopathy such as cerebral infarction is considered to be 10⁷ or more, 10⁶ or more According to the method developed by the inventors, 10⁷ mesenchymal stem cells can be obtained within around 12 days.

The proliferated mesenchymal stem cells may be stored by a technique such as freeze preservation (for example, in a deep freezer at -152°C) until use if required. A culture medium (culture medium for mammalian cells such as RPMI) containing serum (preferably human serum, more preferably autologous serum), dextran, and DMSO is used as freeze preservation liquid for freeze preservation. For example, the cells can be suspended in freeze preservation liquid containing 20.5 mL of common filter-sterilized RPMI, 20.5 mL of autoserum collected from a patient, 5 mL of dextran, and 5 mL of DMSO, and can be freeze-preserved at -150°C. For example, Cryoserv^{™} produced by NIPRO CORPORATION can be used as DMSO, and low molecular dextran L injection produced by Otsuka Pharmaceutical Co., Ltd. can be used as dextran, but the types of DMSO and dextran are not limited to these.

The quality and the function of the mesenchymal stem cells prepared as mentioned above may be confirmed by confirming that a) the mesenchymal stem cells express CX3CL1 by adding a cytokine to the culture containing the mesenchymal stem cells, or b) the mesenchymal stem cells express EGFR and/or ITGA4.

Examples of the "inflammatory cytokine" to be used include TNF-α, INFγ, IL-1, IL-6, IL-8, IL-12, and IL-18. The inflammatory cytokine preferably contains especially TNF-α, INFγ, and IL-6, and a mixture of TNF-α, INFγ, and IL-6 is more preferably used.

The method may further include a step of confirming the existence of any one or more selected from BDNF, VEGF, and HGF in the culture (to which cytokine is not added). It is important to confirm the existence of especially BDNF and/or VEGF, and it is the most important to confirm the existence of BDNF.

If the mesenchymal stem cells express CX3CL1 by adding an inflammatory cytokine, the mesenchymal stem cells can be expected to be excellent in the inflammation modulatory effect (immunomodulatory effect). If 90% or more of the mesenchymal stem cells express EGFR and/or ITGA4, the mesenchymal stem cells can be expected to be excellent in the ability to accumulate at a damaged site. If any of nutritional factors such as BDNF, VEGF, and HGF exists in the culture medium, the culture medium can be expected to contain mesenchymal stem cells having a high neuroprotective effect, and especially the existence of BDNF and/or VEGF, particularly the existence of BDNF may be an important index of MSCs having a high neuroprotective effect. Although the mesenchymal stem cells secrete BDNF, VEGF, and/or HGF without stimulation, for the confirmation of the secretion ability, the secretion from unstimulated cells may be evaluated, and the secretion from cells after inflammatory cytokine stimulation may be evaluated.

The expression of the above-mentioned CX3CL1, EGFR, ITGA4, BDNF, VEGF, and HGF is more preferably evaluated using the expression at the protein level as an index than using the expression at the gene level. In the case of cell surface proteins such as EGFR and ITGA4, it is preferable to measure the cell surface proteins using flow cytometry (FCM) from the viewpoint of simplicity and sensitivity. In the case of secreted proteins such as CX3CL1, BDNF, VEGF, and HGF, it is preferable to use bead assay from the viewpoint of simplicity and sensitivity.

### 2. Pharmaceutical composition for use according to present invention

A pharmaceutical composition for use according to the present invention as defined in the claims is a pharmaceutical composition comprising mesenchymal stem cells for use in a method for preventing in-stent restenosis, wherein the pharmaceutical composition is administered to a patient subjected to stenting as defined in the claims. "In-stent restenosis" means restenosis caused by neointimal hyperplasia in the implanted stent after PTAS. Typically, restenosis is a serious complication which occurs 3 to 6 months after stenting, and leads to the recurrence of cerebral infarction or recurrence of an ischemic attack. The pharmaceutical composition for use according to the present invention as defined in the claims is a pharmaceutical composition for use in a method for preventing this in-stent restenosis as defined in the claims.

The "pharmaceutical for repairing and regenerating tissue" means a pharmaceutical for assisting damaged sites to be restored or for assisting aged sites due to aging to be restored. Examples of the tissue include, but not particularly limited, for example, nervous system tissues including the brain and the spinal cord, the kidney, the pancreas, the liver, the intestines, the stomach, the digestive organs, the lungs, the heart, the spleen, blood vessels, blood, skin, bones, cartilages, teeth, and the prostate gland. Specific examples of diseases and disorders which are targets of the pharmaceutical for repairing and regenerating tissue includes, but not limited to, nephropathy, hepatopathy, pancreatic disorders including diabetes, prostatic hypertrophy, hyperlipemia, higher-order brain dysfunctions including aphasia and dementia, encephalopathy following resuscitation, ischemic heart diseases including angina pectoris and myocardial infarction, ischemic cerebrovascular diseases such as cerebral infarction and arteriosclerosis, and spinal cord injury (refer to International Publication No. WO 2009/034708) .

With regard to the number of mesenchymal stem cells contained in the pharmaceutical composition for use according to the present invention as defined in the claims, an increased number of the cell is more preferable. When time for administration to a patient and time required for culture are taken into consideration, the number is however practically the least amount which exhibits an effect. Therefore, in a preferable aspect of the pharmaceutical composition for use according to the present invention as defined in the claims, the number of mesenchymal stem cells is 5 × 10⁷ or more, more preferably 10⁸ or more, and further preferably 5 × 10⁸ or more.

The pharmaceutical composition for use according to the present invention as defined in the claims is a preparation for parenteral administration, and particularly a preparation for intravenous administration. Examples of dosage forms suitable for parenteral administration include injections such as an injectable solution, an injectable suspension, an injectable emulsion, and injections prepared at the time of use and grafts. The preparation for parenteral administration is in the form of an aqueous or non-aqueous isotonic sterile solution or suspension, and is optionally combined, for example, with pharmacologically acceptable carriers or media, specifically sterile water or physiological saline, culture medium (culture medium such as RPMI used especially for the culture of mammalian cells), physiological buffer solution such PBS, vegetable oil, an emulsifier, a suspending agent, a surfactant, a stabilizer, an excipient, a vehicle, an antiseptic, a binder, and the like into a preparation in a suitable unit dosage form. The preparation may be held by a drug-eluting stent (DES) or the like.

Examples of the solution for injection include physiological saline, culture medium, physiological buffer solution such as PBS, and isotonic solutions containing glucose or other adjuvants, for example, D-sorbitol, D-mannose, D-mannitol, sodium chloride, and the like. The solution for injection may be used in combination with a suitable solubilizing agent, for example, an alcohol, specifically ethanol, polyalcohol, propylene glycol, or polyethylene glycol or a nonionic surfactant, for example, polysorbate 80 or HCO-50, and the like.

Although the first administration time of the pharmaceutical composition for use according to the present invention as defined in the claims is preferably within 180 days, more preferably 0 to 90 days, more preferably 0 to 60 days, and further more preferably 0 to 30 days after PTAS, the time is not limited to these.

### 3. Subject to which pharmaceutical composition for use according to present invention is administered

The pharmaceutical composition for use according to the present invention as defined in the claims is administered to a patient subjected to stenting. "Stenting" is a treatment in which a stent is inserted into a blood vessel, placed and left to reopen the stenosed artery from the inside of the blood vessel, to treat a dissecting lesion such as aortic dissection, or to reinforce the blood vessel in an aneurysm. Examples of the stenting include, but not limited to, methods such as carotid artery stenting (CAS) and percutaneous transluminal angioplasty and stenting (PTAS).

CAS is a therapeutic method in which a stent is placed in a stenosed site of the carotid artery to expand the blood vessel. PTAS is a therapeutic method in which a balloon catheter is inserted into a blood vessel, a balloon is inflated in a stenosed site or an occluded site, the blood vessel is expanded, and a stent is then deployed to expand the blood vessel. The present use in invention is for/patients having the risk of in-stent restenosis and subjected to all types of stenting.

The patients often have atherosclerosis, ischemic heart diseases including myocardial infarction, ischemic cerebrovascular diseases including cerebral infarction and transient ischemic attack (TIA), arteriosclerosis obliterans (ASO), Buerger's disease, arteriosclerotic lesions in blood vessels of the whole body, dissecting lesions of vascular system including a dissecting aneurysm, and an aneurysm, or the like as a primary disease.

### [Ischemic cerebrovascular disease]

Examples of the ischemic cerebrovascular diseases include cerebral infarction (for example, atherothrombotic cerebral infarction, cerebral thrombosis, cerebral embolism, lacunar infarction, BAD (branch atheromatous disease), Trousseau's syndrome, blood coagulation abnormality, arterial dissection, venous infarction, angitis, and an anti-phospholipid antibody syndrome), and transient ischemic attack (TIA).

### [Ischemic heart disease]

Ischemic heart disease is a disease which occurs when the coronary artery is stenosed or occluded due to causes such as arteriosclerosis, and blood does not reach cardiac muscle. Examples thereof include myocardial infarction. Myocardial infarction means a condition in which occlusion or stenosis occurs in the coronary artery blood vessel, which supplies oxygen and nutrition to the heart, the flow rate of blood decreases, and cardiac muscle becomes in an ischemia state, and necroses.

### [Atherosclerosis]

Atherosclerosis means sclerosis of an artery due to lipid abnormalities, diabetes, high blood pressure, smoking, lack of exercise, or the like. Finally, the blood flow of the artery is shut off, so that the shutoff causes cerebral infarction, myocardial infarction, or the like.

### [Dissecting lesion of vascular system]

Dissecting lesion of vascular system means a pathological condition such as a dissecting aneurysm in which, among the intima, the media, and the adventitial coat constituting an arterial wall, the media becomes fragile, and the intima and the adventitial coat dissociate, so that only the adventitial coat remains in the blood vessel at the diseased site, and the blood vessel becomes easy to burst.

### [Aneurysm]

An aneurysm means a pathological condition such as a cerebral aneurysm, an aortic aneurysm, or an abdominal aortic aneurysm, in which the arterial blood vessel wall at the diseased site becomes thin to bulge, resulting in a phyma-like appearance.

### 4. Effect of pharmaceutical composition for use according to present invention

The pharmaceutical composition for use according to the present invention as defined in the claims can prevent in-stent restenosis by reducing inflammatory reaction and inhibiting neointimal hyperplasia. The risk of the recurrence of stroke or the recurrence of ischemic attack accompanying in-stent restenosis can be reduced, and safe stenting can be achieved thereby.

It is known that, as above-mentioned, mesenchymal stem cells have the effects of neuroprotection, neuropoiesis, axon regeneration induction, angiogenesis, the recovery of the blood-brain barrier, remyelination, the preservation of cortex connection, the secretion of neurotrophic factors which impart neural plasticity and remote responsiveness, and the like. In relation to these effects, the inventors have confirmed that administration of mesenchymal stem cells accelerates the regeneration of the damaged site in ischemic angiopathy such as myocardial infarction and cerebral infarction and improves motor dysfunction. The inventors have also confirmed that the administration of mesenchymal stem cells enables the treatment for dementia, cerebral infarction in the chronic phase, spinal cord injury in the chronic phase, neurodegenerative disease, mental disease, higher-order functional disorder, and the like because of the effect of forming synapses and promoting the plasticity in a damaged site (lesion part). Therefore, the pharmaceutical composition for use according to the present invention as defined in the claims can be expected to prevent in-stent restenosis and also exhibit a desirable effect on the above-mentioned primary disease which a patient has or had.

### Examples

### 1. Material and Method

### (1) Preparation of human mesenchymal stem cells

Human MSCs were prepared according to a previous report. Briefly, MSCs (P2) were purchased from LONZA (Walkersville, MD) and diluted with Dulbecco's modified Eagle's medium, and 10% FBS (Life Technologies), 2 mM L-glutamine (Sigma-Aldrich), and 100 U/ml penicillin-streptomycin (Sigma-Aldrich) were added. Then, the MSCs were inoculated into a 150-mm tissue culture dish (AGC TECHNO GLASS CO., LTD.) and incubated in a humidified 5% CO₂ atmosphere at 37°C for several days. When the cells almost reached confluence, the adherent cells were detached with trypsin-EDTA solution (Mediatech, Inc.) and subcultured at 1 × 10⁴ cells/ml. MSCs were thus expanded up to 1 × 10⁸ cells within a relatively short culture period (subculture: 4 times). The expanded MSCs were dissociated, diluted with a storage solution [8 ml of RPMI(Life Technologies), 8 ml of autologous serum obtained from each animal, 2 ml of low molecular dextran L (Otsuka Pharmaceutical Co., Ltd.), and 2 ml of dimethyl sulfoxide (NIPRO CORPORATION)] on the day of stenting, and stored in a freezer (-80°C) (SANYO) until use. A storage solution not containing MSCs was prepared as a placebo. The cryopreserved storage solution containing the MSCs was thawed in a water bath at 37°C at the bedside in the interventional radiology treatment room on the day of administration. Then, 1.0 × 10⁸ cells (survival rate; 99.6 ± 0.5%) or the placebo was intravenously administered to each animal. The phenotype analysis of surface antigens on the MSCs were CD34⁻, CD45⁻, CD73⁺, and CD105⁺.

### (2) Experiment protocol

Experimental protocol is shown in Figure 1. SPF adult mini-pigs weighing 30 to 38 kg (Gottingen minipigs, Oriental Yeast Co., Ltd., Tokyo, Japan) were pretreated with 100 mg/day of aspirin and 15 mg/day of lansoprazole 7 days before stent insertion. Two types of stents: Carotid WALLSTENT^{®} (Boston Scientific Corporation, Natick, MA) and Multi-Link 8^{®} (Abbott Vascular Company, Abbott Park, IL) were deployed respectively on the day of stent insertion. The Placebo or the MSCs (1.0 × 10⁸ cell each) were intravenously administered to all the pigs from the right ear vein under general anesthesia after angiography (DSA) and intravascular ultrasonography (IVUS) 14 days after stent insertion, and the mini-pigs were randomized into two experimental groups, a placebo administration group and a MSC administration group. All pigs were received daily Cyclosporin A (10 mg/kg, orally) was administered to all the pigs daily from 1 day before placebo or MSC administration.

Digital subtraction angiography (DSA) and IVUS were performed immediately after placebo or MSC administration and on day 1, day 7, and day 28. The vessels subjected to PTAS and the contralateral common carotid artery (CCA) which is regarded as control artery were removed for histological analysis after the evaluation of day 28. Physiological parameters were monitored during the procedure of PTAS and the follow-up examination including DSA, and IVUS (Table 1). Experiments using the blood samples before the procedure were also performed at the day of stenting, day 0, day 1, day 10, and day 28 after placebo or MSC administration (Table 2).

### [Table 1]

**Table 1: Physiological parameter**

| | **PTAS (Day -14)** | | | **Day 0** | | | **Day 1** | | | **Day 7** | | | **Day 28** | | |
|---|---|---|---|---|---|---|---|---|---|---|---|---|---|---|---|
| | **Placebo** | **MSC** | **P value** | **Placebo** | **MSC** | **P value** | **Place bo** | **MSC** | **P value** | **Placebo** | **MSC** | **P value** | **Placebo** | **MSC** | **P value** |
| **BW (kg)** | 35 ± 4 | 34 ± 4 | 0.584 | 37 ± 3 | 34 ± 2 | 0.230 | 36 ± 4 | 35 ± 3 | 0.687 | 37 ± 4 | 35 ± 3 | 0.323 | 40 ± 4 | 39 ±3 | 0.641 |
| **BT (°C)** | 36.0 ± 0.5 | 36.5 ± 0.4 | 0.135 | 36.4 ± 0.8 | 36.0 ± 0.3 | 0.668 | 36.7 ± 0.3 | 36.8 ± 0.2 | 0.712 | 36.8 ± 0.3 | 36.5 ± 0.5 | 0.421 | 36.7 ± 0.4 | 36.6 ± 0.6 | 0.630 |
| **sBP(mmHg)** | 98 ± 5 | 103 ± 8 | 0.277 | 128 ± 23 | 120 ± 8 | 0.516 | 110 ± 8 | 117 ± 12 | 0.372 | 113 ± 11 | 110 ± 13 | 0.681 | 113 ± 8 | 114 ± 22 | 0.985 |
| **dBP(mmHg)** | 47 ± 3 | 46 ± 9 | 0.898 | 60 ± 20 | 62 ± 20 | 0.857 | 53 ± 17 | 52 ± 10 | 0.929 | 55± 13 | 53 ± 17 | 0.872 | 60 ± 18 | 49 ± 9 | 0.259 |
| **HR (bpm)** | 99 ± 10 | 98 ± 14 | 0.839 | 110 ± 16 | 103 ± 20 | 0.584 | 97 ± 9 | 100 ± 9 | 0.561 | 98 ± 14 | 98 ± 7 | 0.978 | 93 ± 17 | 100 ±14 | 0.482 |
| **SaO₂ (%)** | 98.8 ± 0.8 | 99.2 ± 0.4 | 0.943 | 98.2 ± 0.4 | 98.6 ± 0.4 | 0.545 | 98.4 ± 1.1 | 99.0 ± 0.7 | 0.347 | 99.0 ± 0.7 | 98.6 ± 1.1 | 0.524 | 99.0 ± 0.4 | 98.6 ± 1.0 | 0.523 |
| **ACT (second)** | 256 ± 25 | 256 ± 62 | 0.985 | 282 ± 51 | 295 ± 67 | 0.818 | 273 ± 57 | 266 ± 59 | 0.857 | 259 ± 45 | 264 ± 78 | 0.455 | 252 ± 26 | 253 ± 36 | 0.850 |
| **pH** | 7.50 ± 0.04 | 7.49 ± 0.02 | 0.717 | 7.50 ± 0.02 | 7.52 ± 0.02 | 0.373 | 7.51 ± 0.04 | 7.52 ± 0.08 | 0.883 | 7.46 ± 0.05 | 7.5 ± 0.1 | 0.913 | 7.49 ± 005 | 7.50 ± 0.05 | 0.652 |
| **pO₂ (mmHg)** | 150 ± 16 | 138 ± 14 | 0.585 | 163 ± 90 | 138 ± 33 | 0.605 | 147 ± 59 | 127 ± 51 | 0.591 | 144 ± 54 | 115 ± 30 | 0.318 | 141 ± 34 | 110 ± 29 | 0.163 |
| **pCO₂ (mmHg)** | 40 ± 2 | 42 ±2 | 0.352 | 39 ± 3 | 39 ± 1 | 0.744 | 38 ± 4 | 39 ± 4 | 0.818 | 46± 6 | 44 ± 6 | 0.476 | 45 ± 2 | 41 ± 6 | 0.236 |
| **HCO₃ (mmol/L)** | 31 ± 1 | 31 ± 2 | 0.837 | 30 ± 3 | 32 ± 2 | 0.422 | 30 ± 2 | 31 ± 3 | 0.492 | 32 ± 3 | 34 ± 3 | 0.458 | 34 ± 3 | 34 ± 4 | 0.857 |

### [Table 2]

**Table 2: Blood sample data**

| | **Stenting (Day -14)** | | | **Day 0 (Infusion of Placebo or MSC)** | | | **Day 1** | | | **Day 7** | | | **Day 28** | | |
|---|---|---|---|---|---|---|---|---|---|---|---|---|---|---|---|
| | **Placebo** | **MSC** | **P value** | **Placebo** | **MSC** | **P value** | **Placebo** | **MSC** | **P value** | **Placebo** | **MSC** | **P value** | **Placebo** | **MSC** | **P value** |
| **WBC (10²/µL)** | 60 ± 13 | 63 ± 10 | 0.791 | 57 ± 20 | 52 ± 13 | 0.677 | 79 ± 40 | 69 ± 17 | 0.612 | 75 ± 29 | 59 ± 12 | 0.273 | 68 ± 15 | 76 ± 11 | 0.406 |
| **RBC (10⁴/µL)** | 435 ± 60 | 489 ± 63 | 0.216 | 472 ± 30 | 487 ± 52 | 0.587 | 415 ± 82 | 443 ± 66 | 0.565 | 397 ± 80 | 449 ± 87 | 0.363 | 477 ± 48 | 525 ± 63 | 0.220 |
| **Hb (g/dL)** | 8.6 ± 1.3 | 9.8 ± 0.7 | 0.107 | 9.7 ± 0.3 | 10.1 ± 0.6 | 0.239 | 8.5 ± 1.4 | 9.3 ± 1.1 | 0.296 | 8.2 ± 1.6 | 9.4 ± 1.3 | 0.229 | 9.9 ± 1.0 | 11.0 ± 1.0 | 0.114 |
| **PLT (10⁴/µL)** | 54 ± 4 | 52 ± 3 | 0.585 | 47± 12 | 43 ± 8 | 0.596 | 38 ± 19 | 39 ± 4 | 0.962 | 63 ± 14 | 65 ±8 | 0.766 | 51 ± 15 | 53 ± 13 | 0.868 |
| **T-Bil (mg/dL)** | 0.3 ± 0.1 | 0.2 ± 0.1 | 0.347 | 0.2 ± 0.1 | 0.2 ± 0.1 | 0.524 | 0.5 ± 0.4 | 0.3 ± 0.1 | 0.329 | 0.4 ± 0.1 | 0.3 ± 0.1 | 0.446 | 0.3 ± 0.1 | 0.3 ± 0.1 | 0.784 |
| **AST (U/L)** | 32 ± 28 | 20 ± 8 | 0.417 | 22 ± 6 | 19 ± 4 | 0.322 | 27± 10 | 30 ± 6 | 0.572 | 24 ± 6 | 24 ± 6 | 0.957 | 21 ± 6 | 23 ± 6 | 0.498 |
| **ALT (U/L)** | 30 ± 19 | 29 ± 3 | 0.880 | 36 ± 13 | 33 ± 6 | 0.654 | 45 ± 22 | 41 ± 4 | 0.641 | 39 ± 19 | 35 ± 9 | 0.679 | 29 ± 8 | 30 ± 9 | 0.775 |
| **LDH (U/L)** | 361 ± 66 | 359 ± 26 | 0.945 | 376 ± 57 | 349 ± 73 | 0.520 | 554 ± 212 | 486 ± 161 | 0.587 | 422 ± 38 | 432 ± 104 | 0.84 | 338 ± 82 | 274 ± 121 | 0.353 |
| **ALP (U/L)** | 150 ± 25 | 162 ± 54 | 0.689 | 153 ± 24 | 161 ± 47 | 0.742 | 272 ± 75 | 180 ± 26 | 0.412 | 128 ± 77 | 150 ± 49 | 0.601 | 153 ± 28 | 152 ± 61 | 0.992 |
| **Alb (g/L)** | 4.4 ± 0.3 | 4.2 ± 0.2 | 0.355 | 4.5 ± 0.5 | 4.4 ± 0.2 | 0.667 | 4.3 ± 0.5 | 4.3 ± 0.1 | 1.000 | 4.4 ± 0.5 | 4.3 ± 0.3 | 0.726 | 4.4 ± 0.5 | 4.5 ± 0.2 | 0.930 |
| **T-cho (mg/dL)** | 76 ± 27 | 76 ± 26 | 0.982 | 92 ± 42 | 74 ± 12 | 0.387 | 60 ± 23 | 60 ± 15 | 0.975 | 74 ± 18 | 77 ± 26 | 0.806 | 84 ± 34 | 71 ± 14 | 0.454 |
| **BUN (mg/dL)** | 4.3 ± 0.8 | 4.8 ± 1.1 | 0.491 | 5.6 ± 2.5 | 5.8 ± 0.4 | 0.825 | 8.3 ± 1.6 | 10.2 ± 2.1 | 0.172 | 5.5 ± 0.7 | 4.8 ± 2.5 | 0.615 | 4.2 ± 1.5 | 5.5 ± 2.4 | 0.325 |
| **Cre (mg/dL)** | 0.6 ± 0.2 | 0.7 ± 0.2 | 0.855 | 0.7 ± 0.2 | 0.8 ± 0.2 | 0.406 | 0.8 ± 0.2 | 0.8 ± 0.2 | 1.000 | 0.6 ± 0.1 | 0.7 ±0.2 | 0.372 | 0.7 ± 0.1 | 0.8 ± 0.3 | 0.654 |
| **Na (mmol/L)** | 144 ± 2 | 144 ± 1 | 0.855 | 145 ± 3 | 144 ± 2 | 0.608 | 145 ± 1 | 145 ± 3 | 0.802 | 145 ± 4 | 144 ± 3 | 0.595 | 144 ± 3 | 144 ± 2 | 1.000 |
| **K(mmol/L)** | 3.6 ± 0.3 | 3.4 ± 0.2 | 0.218 | 3.5 ± 0.4 | 3.6 ± 0.2 | 0.914 | 3.7 ± 0.6 | 3.6 ± 0.1 | 0.638 | 4.0 ± 0.4 | 3.7 ± 0.5 | 0.265 | 4.0 ± 0.2 | 3.9 ± 0.5 | 0.666 |
| **Cl (mmol/L)** | 101 ± 3 | 100 ± 1 | 0.608 | 102 ± 5 | 100 ± 5 | 0.562 | 103 ± 2 | 101 ± 5 | 0.445 | 100 ± 4 | 100 ± 2 | 0.676 | 99 ± 3 | 98 ± 4 | 0.689 |
| **PT (second)** | 12.4 ± 0.5 | 12.1 ± 0.6 | 0.518 | 11.5 ± 2.3 | 12.5 ± 0.9 | 0.406 | 12.8 ± 0.6 | 12.8 ± 1.0 | 0.941 | 12.3 ± 0.7 | 12.5 ± 0.8 | 0.787 | 12.5 ± 0.7 | 11.5 ± 2.0 | 0.314 |
| **APTT (second)** | 22 ± 3 | 24 ± 6 | 0.546 | 25± 1 | 29 ± 8 | 0.327 | 25 ± 6 | 26 ± 6 | 0.939 | 17 ± 8 | 25 ± 9 | 0.204 | 23 ± 6 | 25 ± 6 | 0.525 |
| **Fib (mg/dL)** | 157 ± 76 | 124 ± 31 | 0.398 | 123 ± 67 | 127 ± 36 | 0.914 | 211 ± 48 | 246 ± 21 | 0.176 | 183 ± 48 | 161 ± 76 | 0.584 | 215± 168 | 195 ± 78 | 0.818 |

### (3) Stenting (PTAS)

Stenting was performed under general anesthesia. Animals were fasted for 12 hours prior to surgery. Anesthesia was induced with ketamine (10 mg/kg), dexmedetomidine hydrochloride (0.03 mg/kg), and midazolam hydrochloride (0.18 mg/kg), and maintained via intubation with isoflurane (0.5 to 5%) and all efforts were made to minimize suffering. Lactated Ringer's solution (5 to 10 ml/kg/hour) was intravenously administered during the surgery.

The stenting was performed using a single angiographic system (Infinix Celeve-i; Canon Medical Systems Corporation, Tokyo, Japan). A 5-F sheathless guiding catheter was inserted into the right femoral artery by ultrasonic-guided puncture. Then, the 5-F sheathless guiding catheter was advanced and positioned in the right CCA (Figure 1B, arrows) as a human carotid artery model. Predilation was performed using a 5.0-mm balloon catheter to injure the intimal layer. A Carotid WALLSTENT^{®} (10 × 24 mm) was deployed in the CCA, and postdilation was performed using the 5.0-mm balloon catheter. After stent deployment, IVUS was performed to confirm full expansion of the device. Then, the guiding catheter was positioned in the superficial cervical artery (SCA, Figure 1B, arrow heads) as a human cerebral artery model. The superficial cervical artery was subjected to contrast imaging using a 3.0-mm balloon catheter. A MULTI-LINK8^{®} (3.0 × 23 mm) was deployed in the artery, and predilation was performed using a 3.0-mm balloon catheter. The above was confirmed by IVUS.

All mini-pigs were received 2000 to 3000 units of heparin intravenously to maintain an activated clotting time between 250 and 300 seconds. The animals were placed on a warm water re-circulating heating pad. Physiological parameters including heart rate (HR), systolic arterial blood pressure (sBP), diastolic arterial blood pressure (dBP), body temperature (BT), and oxygen saturation (SaO₂) were monitored constantly throughout the procedure via an oesophageal probe.

### (4) Evaluation of angiographic findings

Angiographic images were obtained using a single angiographic system. This procedure was performed under general anesthesia. A 4-F sheathless guiding catheter was inserted into the femoral artery by ultrasonic-guided paracentesis. Then, the 4-F sheathless guiding catheter was advanced and positioned in the stented artery, and a contrast medium was selectively administered. The quantitative analysis of initial and follow-up angiograms was performed with the NIH Image J plug-in "Neuro J" length analysis tool (version 1.39, National Institutes of Health, Bethesda, Maryland). The stenosis rate is defined as a percentage of the neointimal thickness at the center of the stent: (1 - luminal diameter/stent diameter) × 100. All the initial and follow-up measurements were made in the anterior-posterior projection.

### (5) Evaluation of IVUS findings

IVUS images were obtained using a IVUS catheter (TVC Imaging System, MC8, Infraredx, Inc.) after the acquisition of the angiographic images. A 0.014-inch guide wire was advanced to the distal of the deployed stent using a 4-F sheathless guiding catheter, and the 3.2F rapid exchange catheter was inserted over the wire. The IVUS images were acquired after the administration of 200 µg of nitroglycerine. The stenosis rate is defined as a percentage of the neointimal thickness at the center of the stent: (1 - luminal diameter/stent diameter) × 100.

### (6) Histological findings

The animals were treated for histological analysis at the end point (Figure 1A). Intracardiac perfusion with physiological saline and the 0.1 M phosphate buffer solution (5 liters) was performed. Then, the stented vessel and the contralateral CCA (as a control artery) were removed, and the tissues were fixed with 4% paraformaldehyde. The vessels were embedded in a mixed resin of 2-hydroxyethyl methacrylate and methyl methacrylate monomers. A section at the center of the stent or the normal vessel was cut out with a cement tungsten carbide knife (RM2245, Leica Biosystems Nussloch GmbH, Germany) and stained with hematoxylin and eosin (New Histology Science Laboratory Corporation, Tokyo, Japan). The section was evaluated through an optical microscope (BX51, OLYMPUS CORPORATION).

Histological analysis including the neointimal area and the inflammation score (%) was performed. The neointimal areas were measured around the stent and above the inner membrane. The percentage of the neointimal area was defined as neointimal area/stent area × 100. The extent and the density of inflammatory infiltration in each strut were evaluated using an inflammation score. The grading was as follows: 0: no inflammatory cells surrounding the strut, 1: light noncircumferential lymphohistocytic infiltrate surrounding the strut, 2: localized, moderate to dense cellular aggregate surrounding the strut noncircumferentially, and 3: circumferential dense lymphohistocytic cell infiltration of the strut.

### (7) Statistical analysis

All the statistical analyses were performed using JMP 12.2 of Windows (SAS Institute Inc., Cary, NC). Repeated measures analysis of variance (ANOVA) followed by Sidak post hoc tests was conducted for multiple comparisons of stenosis rates measured in the angiographic images and the IVUS images. The differences between the groups were analyzed by Student's t statistical test. An Error bar indicates an average ± a SD. In the case of p value < 0.050, a measured value was considered to be statistically significant.

### 2. Result

Ten animals underwent implantation of both Carotid WALLSTENT^{®} in CCA, and Multi-Link 8^{®} in SCA. All the animals survived after the treatment and remained healthy until the end point of this study.

### (1) Angiographic findings

The angiographic examination prior to administration of placebo or MSC at day 0 revealed that one SCA stented with ML was occluded, therefore, ten CCA stented with Carotid WALLSTENT^{®} (placebo; n=5, MSC; n=5) and nine SCA stented with Multi-Link 8^{®} (placebo; n=4, MSC; n=5) were analyzed. There was no significant difference in the diameter of both CCA and SCA before stenting between the placebo administration group and MSC administration group respectively (CCA; placebo: 4.82 ± 0.09 mm, MSC: 4.82 ± 0.22 mm, p=0.699, SCA; placebo: 2.83 ± 0.38 mm, MSC: 2.68 ± 0.16 mm, p=0.424). Before administration of placebo or MSC at day 0, there was also no difference in the stenosis rate of stented CCA and SCA between the groups respectively (CCA, Figure 2A, B, E, placebo: 8.4 ± 3.7 %, MSC: 8.8 ± 1.7, p=0.878, SCA, Figure 2C, D, F, placebo: 6.8 ± 2.3%, MSC: 6.8 ± 2.7%, p=0.995).

Although there were no statistical differences in the stenosis rates after placebo or MSC administration, progressive stenosis due to the stenting of the CCAs and the SCAs was observed in both placebo and MSC groups on day 1 and day 7 (Figure 2). The stenosis rates of both CCA and SCA of the MSC administration group were lower than the stenosis rates of the placebo group, respectively, on day 28 after placebo or MSC administration (CCA, Figures 2A, B, and E, placebo: 35.9 ± 8.0, MSC: 34.6 ± 2.7, p = 0.028, SCA, Figures 2C, D, and F, placebo: 24.2 ± 3.7%, MSC: 16.8 ± 4.2%, p = 0.044). Angiographic analysis revealed that the intravenous administration of MSCs prevents neointimal hyperplasia induced by stent implantation in the CCA and the SCA.

### (2) IVUS findings

The ten CCAs stented with the Carotid WALLSTENT^{®} (placebo; N = 5, MSC; n = 5) and the nine SCAs stented with Multi-Link 8^{®} (placebo; n = 4, MSC; N = 5) were analyzed by IVUS. There were no significant differences in the stenosis rates of the CCA and the SCA before placebo or MSC administration at day 0 (CCA, Figures 3A, B, and E, placebo: 8.8 ± 2.1%, MSC: 9.0 ± 2.7, p = 0.892, SCA, Figures 3C, D, and F, placebo: 7.0 ± 2.2%, MSC: 7.4 ± 2.2%, and p = 0.857).

Although there were no statistical differences in the stenosis rates between the two groups at day 1 and day 7 after placebo or MSC administration, the stenosis rates in the stented CCA and SCA in the MSC administration group were significantly lower than those in the placebo administration group at day 28 after placebo or MSC administration (CCA, Figures 3A, B, and E, placebo: 27.6 ± 6.1%, MSC: 18.7 ± 4.1, p = 0.892, SCA, Figures 3C, D, and F, placebo: 19.0 ± 3.3%, MSC: 12.0 ± 3.2%, p = 0.025). IVUS analysis demonstrated that the intravenous administration of MSCs prevented neointimal hyperplasia induced by stent insertion.

### (3) Histological findings

The ten CCAs stented with the Carotid WALLSTENT^{®} (placebo; n = 5, MSC; n = 5) and the nine SCAs stented with the Multi-Link 8^{®} (placebo; n = 4, MSC; n = 5) were analyzed at day 28 after placebo or MSC administration. In the control arteries obtained from the contralateral CCAs, neointimal hyperplasia was not observed in both the placebo administration group and the MSC administration group (Figures 4A and D). In the placebo administration group, neointimal hyperplasia was observed by hematoxylin and eosin (HE) staining in the CCAs stented with the Carotid WALLSTENT^{®}, while in the MSC administration group, less neointimal hyperplasia was observed (Figures 4B, E, and G: placebo: 49.4 ± 10.3, MSC: 38.0 ± 3.8%, p = 0.483). The similar results were observed in the nine SCAs stented with the Multi-Link 8^{®} (Figures 4C, F, and H: placebo: 49.4 ± 9.3%, MSC: 33.1 ± 4.3%, p = 0.010).

HE-stained images at high magnification were used to analyze the infiltration of inflammatory cells in the arterial walls. In the control arteries, inflammatory cells in the arterial walls were not observed in both placebo administration group and MSC administration group (Figures 5A and D). However, infiltration of numerous inflammatory cells were observed surrounding the struts of both Carotid WALLSTENT^{®} (Figure 5B) and Multi-Link 8^{®} (Figure 5C) in the placebo administration group. However, in both Carotid WALLSTENT^{®} (Figure 5E) and Multi-Link 8^{®} (Figure 5F), the degree of the infiltration of inflammatory cells surrounding the struts in the MSC administration group were lower than that in the placebo administration group. As a result of quantitative analysis, the average inflammation scores of the CCA and the SCA in the MSC administration group were significantly lower than the placebo administration group (CCA, Figures 5B, E, and G, placebo: 1.83 ± 0.18, MSC: 1.39 ± 0.14, p = 0.002, SCA, Figures 5C, F, and H, and placebo: 1.89 ± 0.22, MSC: 1.40 ± 0.15, and p = 0.005). Thus, histological analysis demonstrated that the intravenous administration of MSCs prevents neointimal hyperplasia and the infiltration of inflammatory cells into the Carotid WALLSTENT^{®} and the Multi-Link 8^{®}.

### 3. Discussion

In the present investigation, the hypothesis that MSCs inhibited neointimal hyperplasia by reducing inflammatory reaction to a stent strut was verified using the CCAs and the SCAs of the mini-pigs. The diameters of the CCAs and the SCAs of the mini-pigs are similar to those of the CCAs and the MCAs of humans, respectively. Both angiographic and IVUS analysis showed that the intravenous administration of MSCs reduced the degree of the in-stent stenosis in both of the CCA stented with Carotid WALLSTENT^{®} and the SCA stented with Multi-Link 8^{®}. Furthermore, histological examination demonstrated that inflammatory responses surrounding the stent struts were inhibited in both the stented CCA and SCA in the MSC administration group as compared with the placebo administration group.

Although neointimal proliferation in the stented CCA and the SCA was observed in all the animals in the present investigation, the thickness of the neointima in the MSC administration group was thinner than that of the placebo administration group (Figure 4). It has been reported that inflammatory responses after stenting contribute to neointimal formation, which results in in-stent restenosis. Inflammatory factors such as interleukin (IL)-1 and the tumor necrosis factor α (TNF-α) produced by infiltration monocytes and macrophages have an important role in early inflammatory reactions and neointimal proliferation after stenting. In particular, the secretion of IL-1 and TNF-α following an increase in matrix metalloproteinase-9 (MMP-9) continues for at least 3 months after stenting, and the activation of MMP-9 contributes to the degradation of some components in the extracellular matrix and changes the molecule expression on the stented vessel inner wall, which results in neointimal hyperplasia and in-stent restenosis. Thus, the reduction of inflammatory reaction around the stent strut to prevent the activation of MMP-9 might contribute to inhibit the in-stent restenosis.

Previous investigation by the present inventors showed that the intravenous administration of MSCs suppressed the activation of MMP-9. MSCs secrete antiinflammatory factors such as the TNF-α stimulated gene/protein 6 (TSG-6) which inhibits the activation of such MMP-9 through the IL-1 and TNF-α cascade and a transforming growth factor β (TGF-β1) which suppresses MMP-9 activation. Human MSCs used in investigation by the present inventors actually secreted TGF-β1 in vitro in ELISA analysis (93.34 ± 12.65 PG / 1.0 × 10⁴ cells, N = 3). Moreover, recent studies also have indicated that when MSCs are exposed to an inflammation environment, the MSCs can regulate an immune response through the release of various mediators including immunosuppressive molecules, exosomes, and chemokines. Taken together, the intravenous administration of MSCs may inhibit inflammatory reaction around stent struts, which results in prevention of neointimal hyperplasia and in-stent restenosis.

Arteriosclerotic lesions in the major cerebral arteries or the carotid arteries cause stroke, and is responsible for approximately 5 to 20% of ischemic strokes. For these patients with neurological deterioration, stent deployment is performed after the onset of stroke to prevent subsequent neurological deterioration and recurrent ipsilateral stroke. The intravenous administration of MSCs is considered to be a new therapy for ischemic stroke. It is considered that ischemic stroke patients subjected to CAS or PTAS can also be treated by the intravenous administration of MSCs in the future. The intravenous administration of MSCs enables improvement of neurological symptoms and prevention of in-stent stenosis and recurrent stroke. The present study encourages the present protocol which can improve the functional outcome of stroke patients treated by stenting.

Although the intravenous administration of MSCs is known as a pharmaceutical for repairing and regenerating tissue in patients with spinal cord injury in acute phase, the influence on patients subjected to stenting has been unknown. The results of the present study confirmed that MSC intravenous administration to patients treated by stenting has no adverse effect and has the effect of inhibiting intravascular restenosis on the contrary. This enables MSC intravenous administration to patients with acute phase spinal cord injury subjected to stenting.

### Industrial Applicability

The present invention as defined in the claims enables the administration of a pharmaceutical composition comprising mesenchymal stem cells for use in a method for preventing in-stent restenosis, as defined in the claims. The pharmaceutical composition containing MSCs can prevent the progress of neointimal hyperplasia in blood vessels subjected to stenting and prevent restenosis after PTAS.

## Claims

1. A pharmaceutical composition comprising mesenchymal stem cells for use in a method for preventing in-stent restenosis, wherein the pharmaceutical composition is intravenously administered to a patient subjected to stenting, wherein 5 x 10⁷ or more mesenchymal stem cells per dose are administered.

2. The pharmaceutical composition for use according to claim 1, wherein the patient is a patient who suffers from or has suffered from any disease selected from atherosclerosis, ischemic heart diseases including myocardial infarction, ischemic cerebrovascular diseases including cerebral infarction and transient ischemic attack (TIA), arteriosclerosis obliterans (ASO), Buerger's disease, arteriosclerotic lesions in blood vessels of the whole body, vascular dissecting lesions including a dissecting aneurysm, and an aneurysm.

3. The pharmaceutical composition for use according to claim 1 or 2, wherein the stenting is carotid artery stenting (CAS) or percutaneous transluminal angioplasty and stenting (PTAS).

4. The pharmaceutical composition for use according to any one of claims 1 to 3, wherein the mesenchymal stem cells are mesenchymal stem cells derived from bone marrow or blood.

5. The pharmaceutical composition for use according to any one of claims 1 to 4, wherein the mesenchymal stem cells are mesenchymal stem cells derived from bone marrow or blood of the patient.

6. The pharmaceutical composition for use according to any one of claims 1 to 5, wherein the mesenchymal stem cells do not express CD24.

7. The pharmaceutical composition for use according to any one of claims 1 to 6, wherein the pharmaceutical composition is first administered within 180 days after PTAS.

## Patentansprüche

1. Pharmazeutische Zusammensetzung umfassend mesenchymale Stammzellen zur Verwendung in einem Verfahren zur Vorbeugung von In-Stent-Restenose, wobei die pharmazeutische Zusammensetzung einem Patienten, der einer Stentimplantation unterzogen wird ("patient subjected to stenting"), intravenös verabreicht wird, wobei 5 x 10⁷ oder mehr mesenchymale Stammzellen pro Dosis verabreicht werden.

2. Pharmazeutische Zusammensetzung zur Verwendung gemäß Anspruch 1, wobei der Patient ein Patient ist, der an irgendeiner Krankheit leidet oder gelitten hat, ausgewählt aus Atherosklerose, ischämischen Herzerkrankungen einschließlich Myokardinfarkt, ischämischen zerebrovaskulären Erkrankungen einschließlich Hirninfarkt und transitorischer ischämischer Attacke (TIA), Arteriosklerose obliterans (ASO), Buerger-Krankheit, arteriosklerotischen Läsionen in Blutgefäßen des ganzen Körpers, vaskulären Dissektionsläsionen einschließlich einem Dissektionsaneurysma ("dissecting aneurysm") und einem Aneurysma.

3. Pharmazeutische Zusammensetzung zur Verwendung gemäß Anspruch 1 oder 2, wobei die Stentimplantation Carotis-Stentimplantation ("carotid artery stenting"; CAS) oder perkutane transluminale Angioplastie und Stentimplantation ("percutaneous transluminal angioplasty and stanting"; PTAS) ist.

4. Pharmazeutische Zusammensetzung zur Verwendung gemäß irgendeinem der Ansprüche 1 bis 3, wobei die mesenchymalen Stammzellen aus Knochenmark oder Blut abgeleitete mesenchymale Stammzellen sind.

5. Pharmazeutische Zusammensetzung zur Verwendung gemäß irgendeinem der Ansprüche 1 bis 4, wobei die mesenchymalen Stammzellen aus Knochenmark oder Blut des Patienten abgeleitete mesenchymale Stammzellen sind.

6. Pharmazeutische Zusammensetzung zur Verwendung gemäß irgendeinem der Ansprüche 1 bis 5, wobei die mesenchymalen Stammzellen kein CD24 exprimieren.

7. Pharmazeutische Zusammensetzung zur Verwendung gemäß irgendeinem der Ansprüche 1 bis 6, wobei die pharmazeutische Zusammensetzung zunächst innerhalb von 180 Tagen nach PTAS verabreicht wird.

## Revendications

1. Composition pharmaceutique comprenant des cellules souches mésenchymateuses pour une utilisation dans un procédé destiné à prévenir la resténose intra-endoprothèse, dans laquelle la composition pharmaceutique est administrée par voie intraveineuse à un patient ayant été soumis à une pose d'endoprothèse, dans laquelle 5 × 10⁷ ou plus de cellules souches mésenchymateuses par dose sont administrées.

2. Composition pharmaceutique pour une utilisation selon la revendication 1, dans laquelle le patient est un patient qui souffre ou a souffert d'une maladie sélectionnée parmi l'athérosclérose, des maladies cardiaques ischémiques incluant l'infarctus du myocarde, des maladies cérébrovasculaires ischémiques incluant l'infarctus cérébral et l'accident ischémique transitoire (AIT), l'artériosclérose oblitérante (ASO), la maladie de Buerger, des lésions artérioscléreuses dans les vaisseaux sanguins de l'ensemble du corps, des lésions de dissection vasculaire incluant un anévrisme disséquant, et un anévrisme.

3. Composition pharmaceutique pour une utilisation selon la revendication 1 ou la revendication 2, dans laquelle la pose d'endoprothèse est une pose d'endoprothèse de l'artère carotide (CAS) ou une angioplastie transluminale percutanée avec pose d'endoprothèse (PTAS)

4. Composition pharmaceutique pour une utilisation selon l'une quelconque des revendications 1 à 3, dans laquelle les cellules souches mésenchymateuses sont des cellules souches mésenchymateuses dérivées de la moelle osseuse ou du sang.

5. Composition pharmaceutique pour une utilisation selon l'une quelconque des revendications 1 à 4, dans laquelle les cellules souches mésenchymateuses sont des cellules souches mésenchymateuses dérivées de la moelle osseuse ou du sang du patient.

6. Composition pharmaceutique pour une utilisation selon l'une quelconque des revendications 1 à 5, dans laquelle les cellules souches mésenchymateuses n'expriment pas de CD24.

7. Composition pharmaceutique pour une utilisation selon l'une quelconque des revendications 1 à 6, dans laquelle la composition pharmaceutique est administrée pour la première fois dans les 180 jours suivant la PTAS.
